# EUROPÄISCHE PATENTSCHRIFT

(11) **EP 2 701 855 B1**
(45) Veröffentlichungstag und Bekanntmachung des Hinweises auf die Patenterteilung: **26.08.2015**
(21) Anmeldenummer: 12711186.2
(22) Anmeldetag: 30.03.2012
(51) Int. Cl.: B05C 5/02, B05C 7/00

(54) **VORRICHTUNG ZUM BESCHICHTEN EINES SCHLAUCHFÖRMIGEN MEDIZINTECHNISCHEN BAUTEILS MIT KLEBSTOFF**
DEVICE FOR COATING A TUBULAR MEDICAL TECHNICAL COMPONENT WITH ADHESIVE
DISPOSITIF DESTINÉ À REVÊTIR DE COLLE UN ÉLÉMENT TUBULAIRE UTILISÉ EN TECHNIQUE MÉDICALE

(30) Priorität: 27.04.2011 EP 11163835; 27.04.2011 US 201161479417 P
(43) Veröffentlichungstag der Anmeldung: 05.03.2014
(73) Patentinhaber: Fresenius Kabi Deutschland GmbH, 61352 Bad Homburg (DE)
(72) Erfinder: VOGT, Juergen, 36132 Eiterfeld (DE); FRANSEN, Eduard, 36039 Fulda (DE)
(86) Internationale Anmeldenummer: PCT/EP2012/055808
(87) Internationale Veröffentlichungsnummer: WO 2012/146460

(56) Entgegenhaltungen:
- DE-A1- 10 231 231
- US-A- 3 661 621
- US-A- 4 512 947
- US-A- 5 871 584

## Beschreibung

Die Erfindung betrifft eine Vorrichtung zum Benetzen eines medizintechnischen Bauteils mit einem flüssigen Medium nach dem Oberbegriff des Anspruchs 1 und ein Verfahren zum Benetzen eines Bauteils mit einem flüssigen Medium nach dem Oberbegriff des unabhängigen Anspruchs 10.

Eine derartige Vorrichtung zum Benetzen eines medizintechnischen Bauteils mit einem flüssigen Medium weist einen Dispenser zum Aufbringen des flüssigen Mediums auf einen zu benetzenden Abschnitt des Bauteils auf.

Bei einem medizintechnischen Bauteil in diesem Sinne handelt es sich um eine Komponente einer medizintechnischen Apparatur, die mit einem flüssigen Medium benetzt werden soll, um diese Komponente beispielsweise mit einer anderen Komponente klebend zu verbinden. Das medizintechnische Bauteil kann beispielsweise durch einen (flexiblen) Schlauch eines Infusionsbestecks oder ein (rigides) Kunststoffteil einer medizintechnischen Apparatur ausgebildet sein.

Bei dem flüssigen Medium, mit dem das medizintechnische Bauteil zu benetzen ist, kann es sich beispielsweise um einen Klebstoff handeln, der auf das Bauteil aufgetragen und mittels dessen das Bauteil klebend mit einem anderen Bauteil verbunden werden soll. Denkbar ist aber auch, dass es sich bei dem flüssigen Medium um eine andere Flüssigkeit handelt, mit der das Bauteil zu benetzen ist, beispielsweise ein Gleitmittel, insbesondere Isopropanol, zum erleichterten Verbinden zweier Bauteile oder ein medizinisches Silikon zum Silikonisieren eines Bauteils.

Soll herkömmlich ein medizintechnisches Bauteil in Form eines Schlauchs eines Infusionsbestecks klebend mit einem anderen Bauteil, beispielsweise einem Konnektor oder einer Tropfenkammer des Infusionsbestecks, verbunden werden, so wird ein Ende des Schlauchs in eine Petrischale eingetaucht, in der sich ein flüssiges Medium befindet, mittels dessen der Schlauch klebend mit dem Konnektor oder der Tropfenkammer verbunden werden kann.

Dieser herkömmliche Benetzungsvorgang zum Aufbringen des flüssigen Mediums auf ein Ende eines solchen Schlauchs ist prozesstechnisch schwierig.

Unregelmäßigkeiten beim Auftragen des flüssigen Mediums auf das zu benetzende Bauteil können sich beispielsweise ergeben, weil das Bauteil mit dem zu benetzenden Ende nicht bis auf den Grund der Petrischale eingetaucht wird und somit keine vollständige Benetzung des zu benetzenden Abschnitts erfolgt (es ergibt sich ein durch den Bediener bedingter Einflussfaktor).

Zudem sinkt der Pegel des Mediums in der Petrischale über die Zeit, bedingt durch ein Verdunsten des flüssigen Mediums und durch eine Entnahme des Mediums bei Benetzungsvorgängen. Durch unregelmäßiges Nachfüllen des flüssigen Mediums und somit variable Pegelstände des Mediums in der Petrischale kann es zu weiteren Unregelmäßigkeiten in der Benetzung des Bauteils kommen.

Ein weiteres prozesstechnisches Problem bei dem bisherigen Benetzungsvorgang ergibt sich dadurch, dass bei der Benetzung eines Schlauchendes durch Eintauchen in ein flüssiges Medium - aufgrund einer Kapillarwirkung an der Schlauchöffnung des Schlauchendes - nach dem Entnehmen des Schlauchs aus der Petrischale Medium in der eingetauchten Schlauchöffnung verbleibt und zu einem Verschluss der Schlauchöffnung führen kann. Dies soll herkömmlich dadurch vermieden werden, dass das eingetauchte Ende nach dem Entnehmen durch eine sogenannte Schwammscheibe abgetupft wird, so dass überschüssiges Medium aus der Schlauchöffnung entfernt wird. Es ergibt sich hierbei jedoch das Problem, dass es zu einem unzureichenden Entfernen des Mediums aus der Schlauchöffnung kommen kann, wenn das Abtupfen an der Schwammscheibe nicht korrekt durchgeführt wird oder wenn die Schwammscheibe mit dem flüssigen Medium gesättigt ist, so dass sie keine weitere Flüssigkeit aufnehmen kann. Zudem kann es zu Verunreinigungen durch Partikel kommen, die sich an der Schwammscheibe befinden und bei einem Abtupfen an dem Bauteil hängenbleiben.

Darüber hinaus ist der herkömmlich vollständig manuell durchgeführte Benetzungsvorgang dadurch beschränkt, dass bei einem Eintauchen eines Schlauchendes mit einer Schlauchöffnung in ein flüssiges Medium zwangsläufig immer die innere und die äußere Wandung an der Schlauchöffnung des eingetauchten Endes benetzt werden. Eine selektive Benetzung nur der inneren Wandung oder nur der äußeren Wandung ist mit einem simplen Eintauchvorgang nicht zu bewerkstelligen.

Zudem kann es durch eine Verdunstung des flüssigen Mediums aus der verwendeten Petrischale auch zu einer Geruchsbelästigung für einen Bediener kommen.

Weil ein herkömmlicher Benetzungsvorgang dieser Art rein manuell durchgeführt wird, ist er vergleichsweise langsam, ineffizient und zudem fehleranfällig. Obwohl beispielsweise durch eine kontinuierliche Überwachung des Flüssigkeitsstands in einer verwendeten Petrischale, durch sorgfältiges Abtupfen mit der Schwammscheibe, durch ein regelmäßiges Austauschen einer gesättigten Schwammscheibe und durch einen Luftaustausch am Arbeitsplatz ein Teil der bisherigen prozesstechnischen Probleme zuverlässig überwunden werden können, besteht dennoch ein Bedürfnis für einen verbesserten Benetzungsvorgang, bei dem insbesondere die durch die Handhabung eines Bedieners bedingten Einflussfaktoren nach Möglichkeit ausgeschlossen sind.

Aus der US 5,683,541 ist eine Vorrichtung zum Schneiden und Benetzen eines Kunststoffschlauchs bekannt, bei der ein Dispenser zum Aufbringen eines flüssigen Mediums auf ein Ende des Kunststoffschlauchs zum Einsatz kommt. Zum Benetzen wird der Kunststoffschlauch mit einem Ende an den Dispenser angesetzt und wahlweise, abhängig von der Ausgestaltung des Dispensers, an einer inneren Wandung oder einer äußeren Wandung einer Schlauchöffnung benetzt.

Auch bei der aus der US 5,683,541 bekannten Vorrichtung kann es vorkommen, dass nach dem Benetzen eine zu große Menge des flüssigen Mediums in oder an dem zu benetzenden Schlauchende verbleibt, so dass es gegebenenfalls zu einem Verschluss einer Öffnung des Schlauchs kommen kann.

Der vorliegenden Erfindung liegt die Aufgabe zugrunde, eine Vorrichtung und ein Verfahren zum Benetzen eines Bauteils mit einem flüssigen Medium bereitzustellen, mit denen ein Benetzungsvorgang in zumindest teilweise automatisierter Weise durchgeführt Die US 4,512,947 offenbart eine Vorrichtung für das Auftragen eines Lösemittels auf einen Gegenstand, insbesondere eines Schlauches, um an diesem ein anderes Bauteil befestigen zu können.

Der vorliegenden Erfindung liegt die Aufgabe zugrunde, eine Vorrichtung und ein Verfahren zum Benetzen eines Bauteils mit einem flüssigen Medium bereitzustellen, mit denen ein Benetzungsvorgang in zumindest teilweise automatisierter Weise durchgeführt werden kann und dabei das Risiko für einen Verschluss von Öffnungen des Bauteils durch zuviel aufgebrachtes Medium zumindest verringert ist.

Diese Aufgabe wird durch einen Gegenstand mit den Merkmalen des Anspruchs 1 gelöst.

Demnach ist bei einer Vorrichtung zusätzlich eine Saugeinrichtung zum Absaugen zumindest eines Teils des flüssigen Mediums von dem zu benetzenden Abschnitt des Bauteils vorgesehen.

Die vorliegende Erfindung geht von dem Gedanken aus, mittels des Dispensers ein flüssiges Medium einem zu benetzenden Abschnitt eines medizintechnischen Bauteils, beispielsweise eines Kunststoffschlauches, zuzuführen und überflüssiges Medium nach dem Zuführen durch die Saugeinrichtung wieder abzusaugen, so dass überflüssiges Medium nicht auf dem Bauteil verbleibt und nicht zu einem Verschluss von Öffnungen des Bauteils führen kann. Insbesondere wird auf diese Weise einem Kapillareffekt an einer Öffnung eines zu benetzenden Bauteils entgegengewirkt, indem mittels der Saugeinrichtung das zugeführte Medium abgesaugt und damit aus der Bauteilöffnung entfernt wird, so dass das Risiko für einen Verschluss dieser Bauteilöffnung weitestgehend vermieden ist.

Der Dispenser weist vorteilhafterweise zumindest eine Gabeeinrichtung auf, an die das Bauteil in eine Ansetzrichtung ansetzbar ist und die sich zumindest abschnittsweise längs entlang der Ansetzrichtung erstreckt. Zum Benetzen wird das Bauteil an diese Gabeeinrichtung angesetzt und dazu entlang der Ansetzrichtung an die Gabeeinrichtung angesteckt, so dass mittels der Gabeeinrichtung flüssiges Medium hin zu dem zu benetzenden Abschnitt des Bauteils zugeführt und mittels der Saugeinrichtung wieder abgesogen werden kann. Die Gabeeinrichtung weist vorzugsweise ein erstes Ende und ein zweites Ende auf, wobei
- die Gabeeinrichtung ausgebildet ist, im Bereich des ersten Endes das flüssige Medium zum Aufbringen auf den zu benetzenden Abschnitts des Bauteils zuzuführen, und

Dadurch, dass mittels der Gabeeinrichtung das flüssige Medium im Bereich des ersten Endes auf den zu benetzenden Abschnitt des Bauteils aufgetragen wird und die Saugeinrichtung das aufgebrachte flüssige Medium in Richtung des zweiten Endes der Gabeeinrichtung absaugt, wird einerseits erreicht, dass überflüssiges Medium von dem zu benetzenden Bauteil entfernt wird. Ein weiteres Abtupfen des zu benetzenden Bauteils unter Verwendung einer Schwammscheibe, wie bisher üblich, entfällt, so dass auf diese Weise auch das Risiko für eine Verunreinigung durch eingebrachte Partikel vermieden ist. Zudem wird durch das Ansaugen des flüssigen Mediums von dem ersten Ende der Gabeeinrichtung hin zu dem zweiten Ende der Gabeeinrichtung das flüssige Medium an dem zu benetzenden Abschnitt des Bauteils verteilt. Das flüssige Medium fließt, angesaugt durch die Saugeinrichtung, entlang des zu benetzenden Bauteils vom ersten Ende der Gabeeinrichtung hin zum zweiten Ende der Gabeeinrichtung, so dass ein Flüssigkeitsfilm auf den zu benetzenden Abschnitt des Bauteils aufgebracht wird.

Durch die Saugeinrichtung wird darüber hinaus nicht nur überflüssiges Medium abgesaugt, sondern auch durch Verdunsten des flüssigen Mediums entstehende Dämpfe werden entfernt, so dass eine Geruchsbelastung für einen Bediener weitestgehend ausgeschlossen ist.

Die erfindungsgemäße Vorrichtung kann ausgestaltet sein für das Benetzen einer inneren Wandung an einer Öffnung des zu benetzenden Bauteils und/oder einer äußeren Wandung des Bauteils. Hierzu wird jeweils eine besonders gestaltete Gabeeinrichtung verwendet, die der Gestalt des zu benetzenden Abschnitt des Bauteils angepasst ist und wahlweise eine Benetzung einer inneren Wandung und/oder einer äußeren Wandung beispielsweise eines aus Kunststoff hergestellten Schlauchs ermöglicht. Soll ein bestimmtes Bauteil benetzt werden, so wird die dafür angepasste Gabevorrichtung verwendet und an dem Dispenser der Vorrichtung angebracht. Soll ein anderes Bauteil benetzt werden oder soll ein anderer Abschnitt eines Bauteils (beispielsweise anstelle einer äußeren Wandung eines Schlauchendes eine innere Wandung des Schlauchendes) benetzt werden, so wird die Gabevorrichtung ausgetauscht und eine entsprechend angepasste, andere Gabeeinrichtung an den Dispenser angeschlossen und verwendet.

In einer ersten konkreten Ausgestaltung kann die Gabeeinrichtung durch einen Zapfen ausgebildet sein, an den das Bauteil mit einer Öffnung zum abschnittsweisen Benetzen einer inneren Wandung des Bauteils ansetzbar ist. Das beispielsweise als Schlauch ausgebildete Bauteil wird mit seiner Öffnung auf den die Gabeeinrichtung verwirklichenden Zapfen aufgesteckt, so dass der Zapfen in angesetztem Zustand des Bauteils durch zumindest punktuelle Anlage in Kontakt mit dem zu benetzenden Abschnitt der inneren Wandung des Bauteils kommt und das Bauteil an der inneren Wandung der Öffnung benetzen kann.

In anderer Ausgestaltung kann die Gabeeinrichtung auch durch eine Hülse ausgebildet sein, in die das Bauteil mit dem zu benetzenden Abschnitt zum abschnittsweisen Benetzen einer äußeren Wandung des Bauteils einsteckbar ist. Beispielsweise wird ein Schlauch mit einem Ende in die Gabeeinrichtung in Form der Hülse eingesteckt, so dass die Gabeeinrichtung in Form der Hülse in eingestecktem Zustand des Schlauchs flächig an dem zu benetzenden Abschnitt der äußeren Wandung des Schlauchs anliegt.

Sollen gleichzeitig die innere Wandung und die äußere Wandung einer Öffnung z.B. eines Schlauchendes benetzt werden, kann in einer weiteren Ausgestaltung der Dispenser auch zwei Gabeeinrichtungen aufweisen, von denen eine erste Gabeeinrichtung durch einen Zapfen und eine zweite Gabeeinrichtung durch eine den Zapfen umgebende Hülse ausgebildet ist. Zum Benetzen wird das Bauteil zwischen den Zapfen und die Hülse gesteckt, so dass das Bauteil, beispielsweise ein Schlauchende, mit einer Öffnung auf den Zapfen aufgesteckt und gleichzeitig in die Hülse eingesteckt wird. Über die erste Gabeeinrichtung und die zweite Gabeeinrichtung können dann gleichzeitig eine innere Wandung an einer Öffnung des Bauteils und eine äußere Wandung des Bauteils benetzt werden.

In einer konkreten Ausführungsform kann die Gabeeinrichtung einen Kanal zum Zuführen des flüssigen Mediums hin zu dem Bereich des ersten Endes der Gabeeinrichtung aufweisen. Die Gabeeinrichtung weist dabei an einer Wandung, die in angesetztem Zustand des Bauteils dem zu benetzenden Abschnitt des Bauteils zugewandt ist, eine Nut auf, die mit dem Kanal in Strömungsverbindung steht und zumindest abschnittsweise entlang einer um die Ansetzrichtung gerichteten Umfangsrichtung an der Wandung verläuft.

Diese Nut kann beispielsweise eine kreisringförmige Gestalt haben und an der sich um die Ansetzrichtung erstreckenden Wandung der Gabeeinrichtung umlaufen. Mittels der Nut wird dann das flüssige Medium im Bereich des ersten Endes der Gabeeinrichtung kreisringförmig auf das zu benetzende Bauteil aufgebracht. Dadurch, dass mittels der Saugeinrichtung das flüssige Medium von dem ersten Ende hin zu dem zweiten Ende der Gabeeinrichtung gesogen wird, wird das flüssige Medium an dem Bauteil verteilt und ein Flüssigkeitsfilm auf den zu benetzenden Abschnitt des Bauteils aufgebracht, wobei gleichzeitig über die Saugeinrichtung, wie vorangehend bereits beschrieben, überschüssiges Medium abgesaugt und entfernt wird.

Mittels der Vorrichtung kann der Benetzungsvorgang weitestgehend automatisch durchgeführt werden. Zum Benetzen wird das Bauteil mit seinem zu benetzenden Abschnitt an den Dispenser angesetzt, und mittels des Dispensers wird das flüssige Medium auf den zu benetzenden Abschnitt des Bauteils aufgebracht. Mittels der Saugeinrichtung wird dann überflüssiges Medium entfernt und gleichzeitig das Medium so an dem zu benetzenden Abschnitt des Bauteils verteilt, dass sich in gewünschter Weise ein Flüssigkeitsfilm an dem zu benetzendem Abschnitt ergibt. Die Handhabung der Vorrichtung ist dabei einfach und weitestgehend unabhängig von einem Bediener. Um zusätzlich in automatischer Weise den Auftrag des flüssigen Mediums zu steuern, kann eine Dosierungseinrichtung vorgesehen sein, die ausgebildet ist, das flüssige Medium in Abhängigkeit von der Flächengröße und Gestalt des zu benetzenden Abschnitts des Bauteils zu dosieren, so dass für jeden Benetzungsvorgang nicht zu wenig, aber auch nicht zu viel des Mediums bereitgestellt und auf den zu benetzenden Abschnitt des Bauteils aufgebracht wird.

Die Aufgabe wird darüber hinaus auch durch ein Verfahren zum Benetzen eines Bauteils mit einem flüssigen Medium gemäß Anspruch 10 gelost.

Bei dem Verfahren ist vorgesehen, dass
- das Bauteil mit einem zu benetzenden Abschnitt an einen Dispenser einer Vorrichtung zum Benetzen des Bauteils angesetzt wird und
- mit dem Dispenser das flüssige Medium auf den zu benetzenden Abschnitt des Bauteils aufgebracht wird.

Zusätzlich ist vorgesehen, dass mittels einer Saugeinrichtung der Vorrichtung zum Benetzen des Bauteils zumindest ein Teil des flüssigen Mediums von dem zu benetzenden Abschnitt des Bauteils abgesaugt wird.

Die vorangehend für die Vorrichtung beschriebenen Ausgestaltungen können auch für das Verfahren Anwendung finden. Ebenso ergeben sich die beschriebenen Vorteile auch bei dem erfindungsgemäßen Verfahren.

Das Verfahren ist vorteilhafterweise einsetzbar zum Benetzen eines medizintechnischen Bauteils wie eines Schlauchs oder dergleichen, kann grundlegend aber auch zum Benetzen eines beliebigen anderen Bauteils eingesetzt werden.

Der im Rahmen des Verfahrens verwendete Dispenser kann in einer vorteilhaften Ausgestaltung zumindest eine Gabeeinrichtung aufweisen, an die das Bauteil angesetzt wird und die ein erstes Ende und ein zweites Ende aufweist. Mittels der Gabeeinrichtung wird in einem ersten Schritt im Bereich des ersten Endes das flüssige Medium zum Aufbringen auf den zu benetzenden Abschnitt des Bauteils zugeführt, und in einem zweiten Schritt wird mittels der Saugeinrichtung im Bereich des zweiten Endes ein Unterdruck erzeugt, um das flüssige Medium zumindest teilweise aus dem Bereich des ersten Endes hin zu dem zweiten Ende zu saugen. Wie vorangehend beschrieben, kann auf diese Weise zum einen überschüssiges Medium abgesaugt und damit von dem Bauteil nach dem Aufbringen entfernt werden, um einen Verschluss von Öffnungen des Bauteils zu vermeiden. Zudem wird durch die Saugeinrichtung und das Ansaugen des flüssigen Mediums vom ersten Ende der Gabeeinrichtung hin zu dem zweiten Ende der Gabeeinrichtung das flüssige Medium entlang des zu benetzenden Abschnitts des Bauteils verteilt und somit ein Flüssigkeitsfilm auf den zu benetzenden Abschnitt aufgebracht. Die Saugeinrichtung sorgt somit einerseits für die Entfernung überschüssigen Mediums und andererseits für einen gleichmäßigen Auftrag des Mediums auf den zu benetzenden Abschnitt des Bauteils. Zudem können mittels der Saugeinrichtung auch entstehende Dämpfe des Mediums beseitigt werden, um so eine übermäßige Geruchsbelastung für einen Bediener zu vermeiden.

Die Saugeinrichtung saugt das flüssige Medium an, indem sie im Bereich des zweiten Endes der Gabeeinrichtung einen Unterdruck erzeugt. Dieser Unterdruck wird vorzugsweise durch einen Saugimpuls nur für eine kurze Zeitspanne erzeugt, beispielsweise für eine Zeitspanne in der Größenordnung von Millisekunden oder auch Sekunden. Das Benetzen des Bauteils geht somit zeitlich schnell vonstatten, indem nach Ansetzen des Bauteils an den Dispenser unmittelbar das flüssige Medium aufgebracht und mittels der Saugeinrichtung wieder abgesogen wird. Die Gesamtdauer des Benutzungsvorgangs kann deutlich unterhalb von einer Sekunde, vorzugsweise unterhalb von 500 ms liegen, kann aber auch länger sein.

Das Aufbringen des flüssigen Mediums mittels der Gabeeinrichtung und das Absaugen des flüssigen Mediums mittels der Saugeinrichtung können zeitlich nacheinander oder auch gleichzeitig erfolgen.

In einer ersten Variante des Verfahrens wird hierbei zunächst mittels der Gabeeinrichtung das flüssige Medium im Bereich des ersten Endes der Gabeeinrichtung auf das angesetzte Bauteil aufgebracht, um anschließend in einem zweiten Schritt mittels der Saugeinrichtung das flüssige Medium hin zu dem zweiten Ende der Gabeeinrichtung zu saugen und dadurch das Medium an dem zu benetzenden Abschnitt des Bauteils zu verteilen und überschüssiges Medium abzusaugen.

In einer zweiten Variante des Verfahrens kann aber auch das Aufbringen des Mediums mittels der Gabeeinrichtung an dem ersten Ende der Gabeeinrichtung zeitgleich mit dem Absaugen erfolgen. Bei Zuführen des flüssigen Mediums über die Gabeeinrichtung auf den zu benetzenden Abschnitt des Bauteils erzeugt die Saugeinrichtung in dieser Variante bereits einen Unterdruck, so dass das zugeführte Medium unmittelbar in Richtung des zweiten Endes der Gabeeinrichtung gesogen und dadurch entlang des zu benetzenden Abschnitts des Bauteils verteilt wird.

Der der Erfindung zugrunde liegende Gedanke soll nachfolgend anhand der in den Figuren dargestellten Ausführungsbeispiele näher erläutert werden. Es zeigen:
- Fig. 1: eine schematische Darstellung eines Infusionsbestecks;
- Fig. 2: eine schematische, perspektivische Ansicht einer Vorrichtung zum Benetzen eines medizintechnischen Bauteils, in diesem Fall eines Schlauchs eines Infusionsbestecks;
- Fig. 3: eine weitere schematische Ansicht der Vorrichtung zum Benetzen eines medizintechnischen Bauteils;
- Fig. 4: eine schematische Ansicht einer ersten konkreten Ausgestaltung eines Dispensers der Vorrichtung;
- Fig. 5: eine schematische Ansicht einer zweiten konkreten Ausgestaltung des Dispensers der Vorrichtung und
- Fig. 6: eine schematische Ansicht einer dritten konkreten Ausgestaltung eines Dispensers der Vorrichtung.

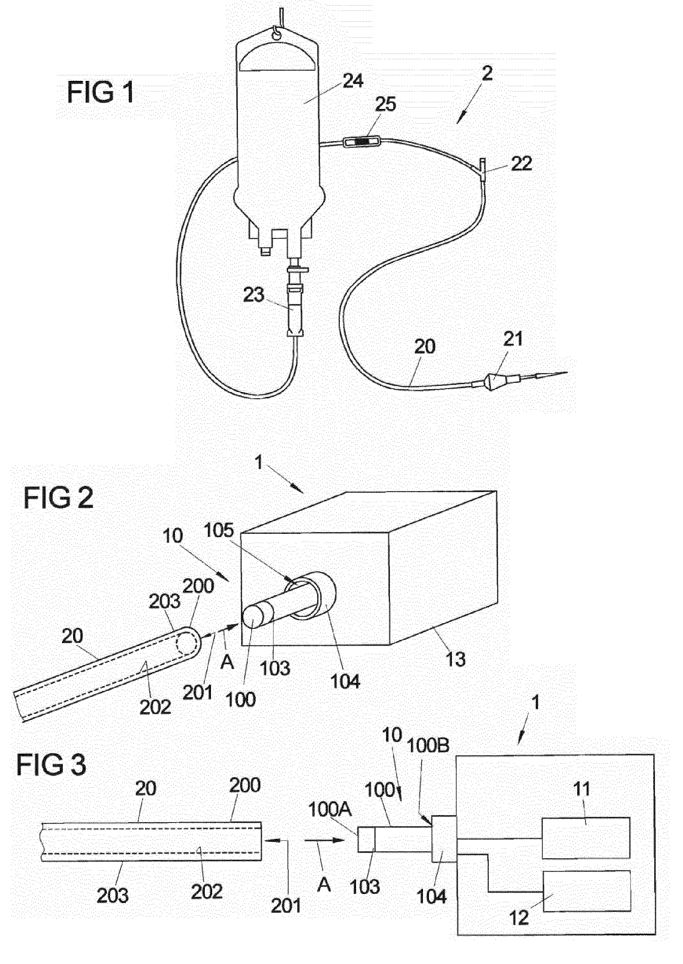

Fig. 1 zeigt in einer schematischen Übersichtsansicht eine medizintechnische Apparatur in Form eines Infusionsbestecks 2, bei der ein Infusionsbeutel 24 (der eine Infusionsflüssigkeit, beispielsweise ein einem Patienten zu verabreichendes Medikament, enthält) über einen aus Kunststoff, insbesondere PVC gefertigten Schlauch 20 mit einem Konnektor 21 verbunden ist. Über den Konnektor 21 kann der Schlauch 20 mit einem Port einer Infusionskanüle verbunden werden, um die in dem Infusionsbeutel 24 enthaltene Flüssigkeit einem Patienten zu verabreichen.

Im Strömungsweg zwischen dem Beutel 24 und dem Konnektor 21 befinden sich, zusätzlich zu dem Schlauch 20, eine Tropfenkammer 23, eine Betätigungseinrichtung 25 und eine Abzweigung 22. Die Tropfenkammer 23 ist an einer Ausflussöffnung des Beutels 24 angeordnet und mit dem Schlauch 20 verbunden. Die Betätigungseinrichtung 25 (beispielsweise ausgestaltet durch einen mechanischen Schieber, mit dem der Schlauch 20 wahlweise geöffnet oder abgeklemmt und somit geschlossen werden kann) ist auf den Schlauch 20 aufgesteckt oder aufgeschoben. Die Abzweigung 22 verbindet zwei Abschnitte des Schlauchs 20 miteinander und dient dazu, beispielsweise einen weiteren Beutel an das Infusionsbesteck 2 anzuschließen.

Zum Herstellen des Infusionsbestecks 2 müssen aufseiten des Herstellers die einzelnen Bauteile 20, 21, 22, 23, 25 miteinander verbunden werden (in der Regel wird der Beutel 24 erst am Orte der Verabreichung an einen Patienten mit der Tropfenkammer 23 verbunden, indem beispielsweise in an sich bekannter Weise die Tropfenkammer 23 mit einem daran angeordneten Dorn in eine entsprechend ausgestaltete Auslassöffnung des Beutels 24 eingesteckt wird). Insbesondere für die Verbindung des Schlauchs 20 mit dem Konnektor 21 einerseits und mit der Tropfenkammer 23 andererseits wird herkömmlich eine klebende Verbindung verwandt, die dadurch hergestellt wird, dass ein Ende des Schlauchs 20 mit einem flüssigen Klebstoffs benetzt und mit dem Konnektor 21 bzw. der Tropfenkammer 23 verbunden wird.

Zum Benetzen eines Bauteils einer medizinischen Apparatur, wie des Schlauchs 20 des Infusionsbestecks 2, sieht die vorliegende Erfindung eine Vorrichtung 1 vor, wie sie in einem Ausführungsbeispiel schematisch in Fig. 2 und 3 dargestellt ist. Die Vorrichtung 1 weist einen Dispenser 10 auf, der an einem Gehäuse 13 der Vorrichtung 1 angeordnet ist und zum Aufbringen eines flüssigen Mediums auf einen zu benetzenden Abschnitt 200 des ein medizintechnisches Bauteil verwirklichenden Schlauchs 20 dient. Die Vorrichtung 1 weist zudem eine Dosierungseinrichtung 11 zum automatischen Dosieren der Menge des aufzubringenden flüssigen Mediums bei einem Benetzungsvorgang und eine Saugeinrichtung 12 zum Absaugen zumindest eines Teils des flüssigen Mediums von dem zu benetzenden Abschnitt 200 des Schlauchs 20 auf.

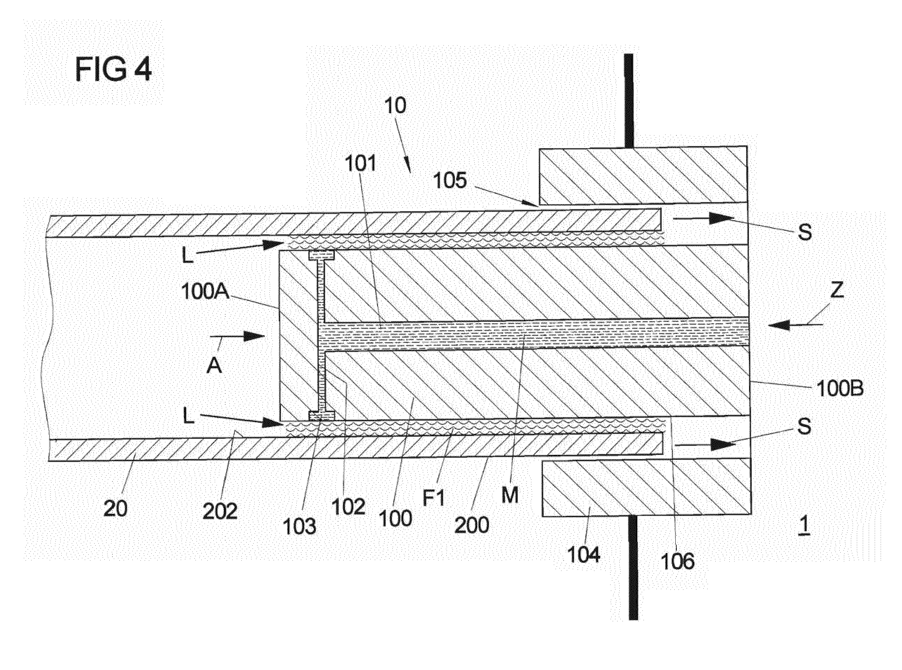

Der Dispenser 10 weist in dem dargestellten Ausführungsbeispiel eine Gabeeinrichtung 100 in Form eines Zapfens auf, wie sie im Einzelnen in Fig. 4 dargestellt ist. Die Gabeeinrichtung 100 in Form des Zapfens steht entlang einer Ansetzrichtung A, in die der Schlauch 20 in Form des Schlauchs an die Gabeeinrichtung 100 anzusetzen ist, von dem Gehäuse 13 der Vorrichtung 1 vor. Die zapfenförmige Gabeeinrichtung 100 ist dabei entsprechend der Formgebung einer inneren Öffnung 201 des Schlauchs 20 kreiszylindrisch ausgebildet und ist zumindest an ihrem dem Gehäuse 13 zugewandten Ende 100B mit radialem Abstand von einem Haltering 104 umgeben. Zwischen dem Haltering 104 und der zapfenförmigen Gabeeinrichtung 100 ist eine Aufnahmeöffnung 105 gebildet, in die der Schlauch 20 mit der Öffnung 201 eingesteckt werden kann.

Zum Benetzen wird der Schlauch 20 mit seinem zu benetzenden Abschnitt 200 (angeordnet an einem Schlauchende) auf die Gabeeinrichtung 100 aufgesteckt, so dass die Gabeeinrichtung 100 in die Öffnung 201 eingeführt wird und an der inneren Wandung 202 des Schlauchs 20 zu liegen kommt. Der Schlauch 20 wird dabei in der Aufnahmeöffnung 105 zwischen der Gabeeinrichtung 100 und dem kreiszylindrischen Haltering 104, der die Gabeeinrichtung 100 ringförmig umgibt, gehalten.

Wie aus der schematischen Detaildarstellung gemäß Fig. 4 ersichtlich, weist die Gabeeinrichtung 100 in Form des kreiszylindrischen Zapfens einen inneren Kanal 101 auf, der sich entlang der Ansetzrichtung A in der Gabeeinrichtung 100 erstreckt. Über einen quer zur Ansetzrichtung A verlaufenden, weiteren Kanal 102 ist dieser Kanal 101 in Strömungsverbindung mit einer Nut 103, die an einem von dem Gehäuse 13 abliegenden, ersten Ende 100A der Gabeeinrichtung 100 angeordnet ist und kreisringförmig an einer der inneren Wandung 202 des Schlauchs 20 zugewandten, zylindermantelförmigen äußeren Wandung 106 der zapfenförmigen Gabeeinrichtung 100 umläuft.

Im Rahmen eines Benetzungsvorgangs wird zunächst der Schlauch 20 mit seinem zu benetzenden Abschnitt 200 in die Aufnahmeöffnung 105 eingesteckt und dadurch die Gabeeinrichtung 100 in die Öffnung 201 des Schlauchs 20 eingeschoben. Mittels der Dosierungseinrichtung 11 (siehe Fig. 3) wird über die Kanäle 101, 102 eine vorbestimmte Dosis des flüssigen Mediums M in eine Zuführrichtung Z hin zu dem ersten Ende 100A der Gabeeinrichtung 100 geleitet und über die Nut 103 kreisringförmig auf die innere Wandung 202 des Schlauchs 20 aufgebracht.

Gleichzeitig mit diesem Aufbringen oder anschließend nach dem Aufbringen wird mittels der Saugeinrichtung 12 (siehe Fig. 3) im Bereich eines dem Gehäuse 13 zugewandten, zweiten Endes 100B der Gabeeinrichtung 100 in dem Raum der Aufnahmeöffnung 105 zwischen der Gabeeinrichtung 100 und dem Haltering 104 ein Unterdruck erzeugt, mittels dessen das im Bereich des ersten Endes 100A der Gabeeinrichtung 100 auf die innere Wandung 202 des Schlauchs 20 aufgebrachte Medium M entlang einer Saugrichtung S hin zu dem zweiten Ende 100B der Gabeeinrichtung 100 gesogen wird, so dass das flüssige Medium M entlang der inneren Wandung 202 des Schlauchs 20 fließt und dadurch einen Flüssigkeitsfilm F1 an der inneren Wandung 202 des Schlauches 20 bildet.

Durch das Ansaugen des flüssigen Mediums M mittels der Saugeinrichtung 12 wird zum einen erreicht, dass überschüssiges Medium M abgesaugt und entfernt wird. Das Risiko für einen Verschluss des Schlauchs 20 durch das flüssige Medium M aufgrund eines Kapillareffekts wird dadurch weitestgehend ausgeschlossen.

Zum anderen wird mittels des Ansaugens das Medium M gleichmäßig zur Bildung eines Flüssigkeitsfilms F1 auf der inneren Wandung 202 im Bereich des zu benetzenden Abschnitts verteilt, so dass sichergestellt ist, dass der Schlauch 20 im Bereich seines zu benetzenden Abschnitts 200 gleichmäßig und entlang der gewünschten Länge mit einem Flüssigkeitsfilm F1 bedeckt ist.

Zudem wird durch das Ansaugen des flüssigen Mediums M auch das Risiko für eine Verunreinigung des Flüssigkeitsfilms F1 zumindest reduziert. Denn mit dem Ansaugen des flüssigen Mediums M in Richtung des zweiten Endes 100B der Gabeeinrichtung 100 wird im Bereich des ersten Endes 100A der Gabeeinrichtung 100 eine nachströmende Luftströmung L angesogen, die zusammen mit dem Medium M für eine Durchströmung zwischen der inneren Wandung 202 des Schlauchs 20 und der Gabeeinrichtung 100 hin zu dem zweiten Ende 100B der Gabeeinrichtung 100 führt und etwaig vorhandene Partikel aus diesem Bereich entfernt.

Der von der Saugeinrichtung 12 erzeugte Unterdruck wird vorzugsweise nur für eine kurze Zeitspanne nach Art eines Saugimpulses mit einer Länge von einigen Millisekunden (z.B. 100 Millisekunden) oder auch Sekunden angelegt. Dieser Saugimpuls kann gleichzeitig mit dem Zuführen des Mediums M in die Zuführrichtung Z über die Kanäle 101, 102 oder auch nach dem Zuführen angelegt werden. Insgesamt ergibt sich ein zeitlich kurzer Benetzungsvorgang, der in einer Zeitspanne von unter einer Sekunde, beispielsweise unter 0,5 s abgeschlossen sein kann.

Die Beschaffenheit des Flüssigkeitsfilms, insbesondere die im Flüssigkeitsfilm F1 enthaltene Menge des Mediums M und damit einhergehend die Dicke des Flüssigkeitsfilms F1, kann eingestellt werden durch das Dosiervolumen über die Dosierungseinrichtung 11 und die Saugzeit und den Unterdruck über die Saugeinrichtung 12.

Nach erfolgtem Benetzungsvorgang wird der Schlauch 20 mit seinem Ende 200 aus dem Dispenser 10 gezogen und mit dem benetzten Abschnitt 200 an das zu verbindende Bauteil, beispielsweise den Konnektor 21 (siehe Fig. 1), angesetzt, um dieses klebend mit dem Schlauch 20 zu verbinden.

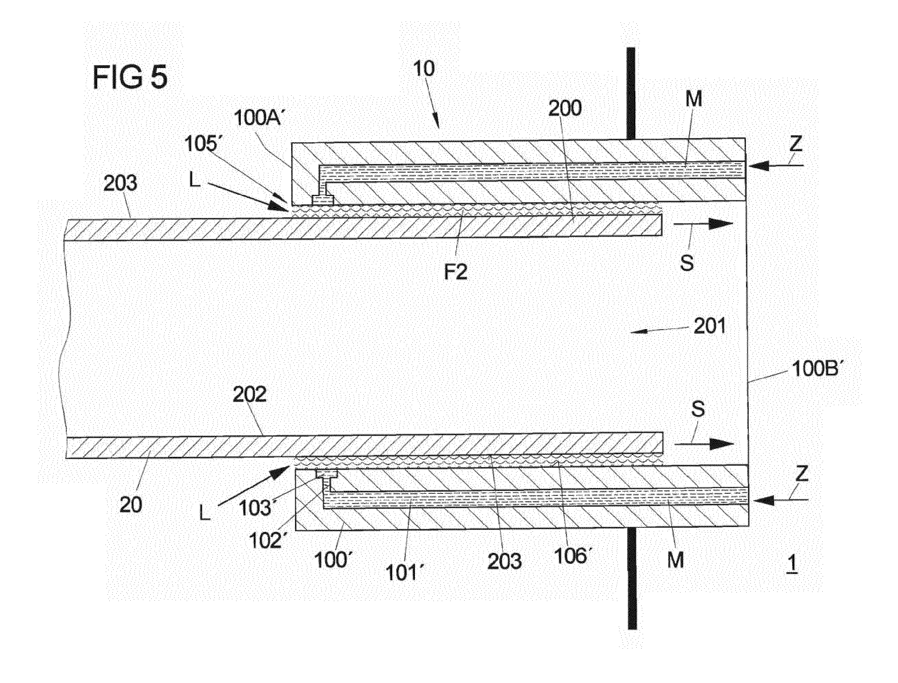

Bei einem schematisch in Fig. 5 dargestellten Ausführungsbeispiel weist der Dispenser 10 eine Gabeeinrichtung 100' in Form einer Hülse auf, die in ihrer Grundform kreiszylindrisch ausgebildet ist und eine Aufnahmeöffnung 105' zum Einstecken des zu benetzenden Abschnitts 200 des Schlauchs 20 aufweist. Die Gabeeinrichtung 100' weist wiederum einen Kanal 101' oder mehrere Kanäle 101' auf, der bzw. die über einen oder mehrere Kanäle 102' mit einer ringförmig an einer inneren Wandung 106' der hülsenförmigen Gabeeinrichtung 100' umlaufenden, kreisförmigen Nut 103' in Strömungsverbindung stehen.

Die Gabeeinrichtung 100' dient zum Benetzten der äußeren Wandung 203 des Schlauchs 20 im Bereich von dessen Schlauchende. Hierzu wird der Schlauch 20 mit seinem Ende in die Aufnahmeöffnung 105' der Gabeeinrichtung 100' eingesteckt, und über die Kanäle 101', 102' wird das Medium M in Richtung eines ersten Endes 100A' der Gabeeinrichtung 100' zugeführt und über die ringförmige Nut 103' auf die äußere Wandung 203 des Schlauchs 20 aufgebracht. Über die Saugeinrichtung 12 (siehe Fig. 3) der Vorrichtung 1 wird dann, gleichzeitig mit dem Aufbringen oder anschließend nach dem Aufbringen, ein Unterdruck im Bereich eines zweiten Endes 100B' der Gabeeinrichtung 100' erzeugt, um auf diese Weise das im Bereich des ersten Endes 100A' auf die äußere Wandung 203 des Schlauchs 20 aufgebrachte Medium M hin zu dem zweiten Ende 100B' der Gabeeinrichtung 100' zu saugen und auf diese Weise einen Flüssigkeitsfilm F2 an der äußeren Wandung 203 des Schlauchs 20 zu erzeugen und überschüssiges Medium M abzusaugen.

Um in wirkungsvoller Weise einen Unterdruck im Bereich des zweiten Endes 100B' der Gabeeinrichtung 100' zu erzeugen, kann zusätzlich ein zentraler Zapfen vorgesehen sein, der innerhalb der Aufnahmeöffnung 105' radial mit Abstand zu der inneren Wandung 106' der Gabeeinrichtung 100' lagefest zu der Gabeeinrichtung 100' angeordnet ist und in eingestecktem Zustand des Schlauchs 20 in die Öffnung 201 des Schlauchs 20 eingreift.

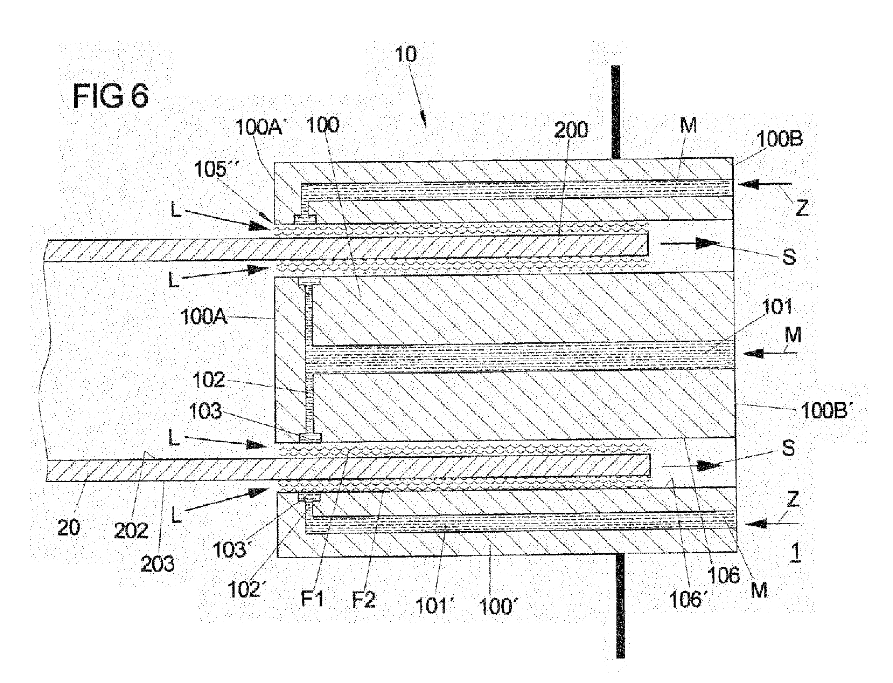

Bei einem weiteren, in Fig. 6 dargestellten Ausführungsbeispiel ist sowohl eine Gabeeinrichtung 100 nach Art eines inneren Zapfens als auch eine Gabeeinrichtung 100' nach Art einer äußeren Hülse vorgesehen. Mittels der beiden Gabeeinrichtungen 100, 100' kann sowohl ein Flüssigkeitsfilm F1 auf die innere Wandung 202 als auch ein Flüssigkeitsfilm F2 auf die äußere Wandung 203 des Schlauches 20 aufgebracht werden, um den Schlauch 20 im Bereich seines Endes sowohl innen als auch außen zu benetzen. Der Benetzungsvorgang ist hierbei analog wie vorangehend beschrieben, so dass auf das oben Ausgeführte verwiesen werden soll.

Um unterschiedliche medizintechnische Bauteile, beispielsweise Schläuche 20 unterschiedlicher Art, beispielsweise unterschiedlichen Durchmessers, benetzen zu können, können unterschiedliche Gabeeinrichtungen 100, 100' mit unterschiedlicher konkreter Formgestaltung verwendet werden, wobei eine bestimmte Gabeeinrichtung 100, 100' in ihrer geometrischen Form dem zu benetzenden Bauteil angepasst ist. Sollen mit einer Vorrichtung 1 unterschiedliche Bauteile benetzt werden, so wird abhängig von dem jeweils konkret zu benetzenden Bauteil der Dispenser 10 mit einer geeigneten Gabeeinrichtung 100, 100' bestückt, wobei die Gabeeinrichtung 100, 100' vorteilhafterweise lösbar und austauschbar sein kann, um in weiteren Arbeitsgängen andere Bauteile benetzen zu können.

Die Vorrichtung 1 kann so beschaffen und ausgestaltet sein, dass der Benetzungsvorgang automatisch eingeleitet wird, sobald das zu benetzende Bauteil an dem Dispenser 10 angesetzt wird. Hierzu kann eine geeignete Detektionsvorrichtung vorgesehen sein, die erkennt, ob das Bauteil in korrekter Weise an den Dispenser 10 angesetzt worden ist, um daraufhin in automatischer Weise den Benetzungsvorgang durch Aufbringen des Mediums M mittels der Dosierungsvorrichtung 11 und Absaugen mittels der Saugeinrichtung 12 durchzuführen.

Der der Erfindung zugrunde liegende Gedanke ist nicht auf die vorangehend geschilderten Ausführungsbeispiele beschränkt, sondern lässt sich auch bei grundsätzlich anders gearteten Ausführungsformen verwirklichen. Insbesondere können unterschiedlichste medizinische Bauteile in der beschriebenen Weise mittels der beschriebenen Vorrichtung benetzt werden, um auf diese Weise mit einem Flüssigkeitsfilm überzogen zu werden. Die Erfindung ist nicht auf die Benetzung von Schläuchen zur Verwendung in einem Infusionsbesteck beschränkt.

Zudem ist die Erfindung nicht auf das Benetzen mit einem flüssigen Medium zur klebenden Verbindung eines Bauteils mit einem anderen beschränkt. Eine Vorrichtung der geschilderten Art kann grundsätzlich auch dazu verwendet werden, ein flüssiges Medium in Form eines Gleitmittels, beispielsweise Isopropanol, zum erleichterten Verbinden zweier Bauteile oder in Form eines medizinischen Silikons zum Silikonisieren eines Bauteils aufzubringen. Auch andere Einsatzmöglichkeiten, bei denen ein Flüssigkeitsfilm auf ein medizintechnisches Bauteil aufgebracht werden soll, sind denkbar.

### Bezugszeichenliste

- 1: Vorrichtung
- 10: Dispenser
- 100, 100': Gabeeinrichtung
- 100A, 100B, 100A', 100B': Ende
- 101, 102, 101', 102': Kanal
- 103, 103': Umlaufende Nut
- 104: Haltering
- 105, 105', 105": Aufnahmeöffnung
- 106, 106': Wandung
- 11: Dosierungseinrichtung
- 12: Saugeinrichtung
- 13: Gehäuse
- 2: Infusionsbesteck
- 20: Bauteil (Schlauch)
- 200: Zu benetzender Abschnitt
- 201: Öffnung
- 202: Innere Wandung
- 203: Äußere Wandung
- 21: Konnektor
- 22: Abzweigung
- 23: Tropfenkammer
- 24: Infusionsbeutel
- 25: Betätigungseinrichtung
- A: Ansetzrichtung
- F1, F2: Flüssigkeitsfilm
- L: Luftströmung
- M: Medium
- S: Saugrichtung
- Z: Zuführrichtung

## Patentansprüche

1. Vorrichtung (1) zum Benetzen eines medizintechnischen Bauteils (20) mit einem flüssigen Medium (M), mit
- einem Dispenser (10) zum Aufbringen des flüssigen Mediums (M) auf einen zu benetzenden Abschnitt (200) des Bauteils (20) und
- einer Saugeinrichtung (12) zum Absaugen zumindest eines Teils des flüssigen Mediums (M) von dem zu benetzenden Abschnitt (200) des Bauteils (20),
**dadurch gekennzeichnet,**
**dass** der Dispenser (10) zumindest eine Gabeeinrichtung (100, 100') aufweist, an die ein Bauteil (20) in eine Ansetzrichtung (A) ansetzbar ist und die sich zumindest abschnittsweise längs entlang der Ansetzrichtung (A) erstreckt, und die Gabeeinrichtung (100, 100') ein erstes Ende (100A, 100A') und ein zweites Ende (100B, 100B') aufweist, wobei
- die Gabeeinrichtung (100, 100') ausgebildet ist, im Bereich des ersten Endes (100A, 100A') das flüssige Medium (M) zum Aufbringen auf den zu benetzenden Abschnitt (200) des Bauteils (20) zuzuführen, und
- die Saugeinrichtung (12) ausgebildet ist, im Bereich des zweiten Endes (100B, 100B') einen Unterdruck zu erzeugen, um das flüssige Medium (M) zumindest teilweise aus dem Bereich des ersten Endes (100A, 100A') hin zu dem zweiten Ende (100B, 100B') zu saugen, so dass das flüssige Medium (M), angesaugt durch die Saugeinrichtung (12), entlang des zu benetzenden Bauteils (20) vom ersten Ende (100A, 100A') der Gabeeinrichtung (100, 100') hin zum zweiten Ende 100B, 100B') der Gabeeinrichtung (100, 100') fließt und ein Flüssigkeitsfilm auf den zu benetzenden Abschnitt (200) des Bauteils (20) aufgebracht wird.

2. Vorrichtung (1) nach Anspruch 1, **dadurch gekennzeichnet, dass** die Gabeeinrichtung (100) durch einen Zapfen ausgebildet ist, an den das Bauteil (20) mit einer Öffnung (201) zum abschnittsweisen Benetzen einer inneren Wandung (202) des Bauteils (20) ansetzbar ist.

3. Vorrichtung (1) nach Anspruch 2, **dadurch gekennzeichnet, dass** die Gabeeinrichtung (100) in Form des Zapfens in angesetztem Zustand des Bauteils (20) mit dem zu benetzenden Abschnitt (200) der inneren Wandung (202) des Bauteils (20) in Kontakt ist.

4. Vorrichtung (1) nach Anspruch 1, **dadurch gekennzeichnet, dass** die Gabeeinrichtung (100') durch eine Hülse ausgebildet ist, in die das Bauteil (20) mit dem zu benetzenden Abschnitt (200) zum abschnittsweisen Benetzen einer äußeren Wandung (203) des Bauteils (20) einsteckbar ist.

5. Vorrichtung (1) nach Anspruch 4, **dadurch gekennzeichnet, dass** die Gabeeinrichtung (100) in Form der Hülse in eingestecktem Zustand des Bauteils (20) flächig an dem zu benetzenden Abschnitt (200) der äußeren Wandung (203) des Bauteils (20) anliegt.

6. Vorrichtung (1) nach einem der vorangehenden Ansprüche, **dadurch gekennzeichnet, dass** der Dispenser zwei Gabeeinrichtungen (100, 100') aufweist, von denen
- eine erste Gabeeinrichtung (100) durch einen Zapfen ausgebildet ist, an den das Bauteil (20) mit einer Öffnung (201) zum abschnittsweisen Benetzen einer inneren Wandung (202) des Bauteils (20) ansetzbar ist, und
- eine zweite Gabeeinrichtung (100') durch eine Hülse ausgebildet ist, in die das Bauteil (20) mit dem zu benetzenden Abschnitt (200) zum abschnittsweisen Benetzen einer äußeren Wandung (203) des Bauteils (20) einsteckbar ist.

7. Vorrichtung (1) nach einem der vorangehenden Ansprüche, **dadurch gekennzeichnet, dass** die zumindest eine Gabeeinrichtung (100, 100') einen Kanal (101, 102, 101', 102') zum Zuführen des flüssigen Mediums (M) hin zu dem Bereich des ersten Endes (100A, 100A') der Gabeeinrichtung (100, 100') aufweist, wobei die Gabeeinrichtung (100, 100') an einer Wandung (106, 106'), die in angesetztem Zustand des Bauteils (20) dem zu benetzenden Abschnitt (200) des Bauteils (20) zugewandt ist, eine Nut (103, 103') aufweist, die mit dem Kanal (101, 102, 101', 102') in Strömungsverbindung steht und zumindest abschnittsweise entlang einer um die Ansetzrichtung (A) gerichteten Umfangsrichtung an der Wandung (106, 106') verläuft.

8. Vorrichtung (1) nach Anspruch 7, **dadurch gekennzeichnet, dass** die Nut (103, 103') kreisringförmig an der sich um die Ansetzrichtung (A) erstreckenden Wandung (106, 106') der Gabeeinrichtung (100, 100') umläuft.

9. Vorrichtung (1) nach einem der vorangehenden Ansprüche, **gekennzeichnet durch** eine Dosierungseinrichtung (11) zum automatischen Dosieren der Menge des über den Dispenser (10) zuzuführenden flüssigen Mediums (M).

10. Verfahren zum Benetzen eines Bauteils (20) mit einem flüssigem Medium (M), bei dem
- das Bauteil (20) mit einem zu benetzenden Abschnitt (200) an einen Dispenser (10) einer Vorrichtung (1) zum Benetzen des Bauteils (20) angesetzt wird,
- mit dem Dispenser (10) das flüssige Medium (M) auf den zu benetzenden Abschnitt (200) des Bauteils (20) aufgebracht wird und
- wobei der Dispenser (10) zumindest eine Gabeeinrichtung (100, 100') aufweist, an die das Bauteil (20) in eine Ansetzrichtung (A) angesetzt wird und die sich zumindest abschnittsweise längs entlang der Ansetzrichtung (A) erstreckt, und die Gabeeinrichtung (100, 100') ein erstes Ende (100A, 100A') und ein zweites Ende (100B, 100B') aufweist, wobei
- mittels der Gabeeinrichtung (100, 100') im Bereich des ersten Endes (100A, 100A') das flüssige Medium (M) zum Aufbringen auf den zu benetzenden Abschnitt (200) des Bauteils (20) zugeführt wird, **dadurch gekennzeichnet, dass** mittels einer Saugeinrichtung (12) der Vorrichtung (1) zum Benetzen des Bauteils (20) zumindest ein Teil des flüssigen Mediums (M) von dem zu benetzenden Abschnitt (200) des Bauteils (20) abgesaugt wird, und
- mittels der Saugeinrichtung (12) im Bereich des zweiten Endes (100B, 100B') ein Unterdruck erzeugt wird, um das flüssige Medium (M) zumindest teilweise aus dem Bereich des ersten Endes (100A, 100A') hin zu dem zweiten Ende (100B, 100B') zu saugen, so dass das flüssige Medium (M), angesaugt durch die Saugeinrichtung (12), entlang des zu benetzenden Bauteils (20) vom ersten Ende (100A, 100A') der Gabeeinrichtung (100, 100') hin zum zweiten Ende 100B, 100B') der Gabeeinrichtung (100, 100') fließt und ein Flüssigkeitsfilm auf den zu benetzenden Abschnitt (200) des Bauteils (20) aufgebracht wird.

11. Verfahren nach Anspruch 10, **dadurch gekennzeichnet, dass**
- mittels der Gabeeinrichtung (100, 100') in einem ersten Schritt im Bereich des ersten Endes (100A, 100A') das flüssige Medium (M) zum Aufbringen auf den zu benetzenden Abschnitt (200) des Bauteils (20) zugeführt wird und
- mittels der Saugeinrichtung (12) in einem zweiten Schritt im Bereich des zweiten Endes (100B, 100B') ein Unterdruck erzeugt wird, um das flüssige Medium (M) zumindest teilweise aus dem Bereich des ersten Endes (100A, 100A') hin zu dem zweiten Ende (100B, 100B') zu saugen.

## Claims

1. Apparatus (1) for coating a medical-technical component (20) with a liquid medium (M), having
- a dispenser (10) for applying the liquid medium (M) to a section (200) to be coated of the component (20), and
- a suction device (12) for suctioning off at least a part of the liquid medium (M) from the section (200) to be coated of the component (20),
**characterized**
**in that** the dispenser (10) has at least one dispensing device (100, 100') onto which a component (20) can be mounted in a mounting direction (A) and which, at least in sections, extends longitudinally along the mounting direction (A), and the dispensing device (100, 100') has a first end (100A, 100A') and a second end (100B, 100B'), wherein
- the dispensing device (100, 100') is designed such that, in the region of the first end (100A, 100A'), it supplies the liquid medium (M) for applying to the section (200) to be coated of the component (20), and
- the suction device (12) is designed so as to generate a negative pressure in the region of the second end (100B, 100B') in order to suction the liquid medium (M) at least partially from the region of the first end (100A, 100A') to the second end (100B, 100B'), such that the liquid medium (M), suctioned by the suction device (12), flows along the component (20) to be coated from the first end (100A, 100A') of the dispensing device (100, 100') to the second end (100B, 100B') of the dispensing device (100, 100'), and a liquid film is applied to the section (200) to be coated of the component (20).

2. Apparatus (1) according to Claim 1, **characterized in that** the dispensing device (100) is formed by a peg onto which the component (20) can be mounted by way of an opening (201) in order for an internal wall (202) of the component (20) to be coated at least in sections.

3. Apparatus (1) according to Claim 2, **characterized in that** the dispensing device (100) in the form of the peg is in contact with the section (200) to be coated of the internal wall (202) of the component (20) when the component (20) is in the mounted state.

4. Apparatus (1) according to Claim 1, **characterized in that** the dispensing device (100') is formed by a sleeve into which the component (20) can be inserted by way of the section (200) to be coated in order for an external wall (203) of the component (20) to be coated at least in sections.

5. Apparatus (1) according to Claim 4, **characterized in that** the dispensing device (100) in the form of the sleeve bears areally against the section (200) to be coated of the external wall (203) of the component (20) when the component (20) is in the inserted state.

6. Apparatus (1) according to one of the preceding claims, **characterized in that** the dispenser has two dispensing devices (100, 100'), of which
- a first dispensing device (100) is formed by a peg onto which the component (20) can be mounted by way of an opening (201) in order for an internal wall (202) of the component (20) to be coated at least in sections, and
- a second dispensing device (100') is formed by a sleeve into which the component (20) can be inserted by way of the section (200) to be coated in order for an external wall (203) of the component (20) to be coated at least in sections.

7. Apparatus (1) according to one of the preceding claims, **characterized in that** the at least one dispensing device (100, 100') has a duct (101, 102, 101', 102') for the supply of the liquid medium (M) to the region of the first end (100A, 100A') of the dispensing device (100, 100'), wherein, on a wall (106, 106') which, in the mounted state of the component (20), faces toward the section (200) to be coated of the component (20), the dispensing device (100, 100') has a groove (103, 103') which is connected in terms of flow to the duct (101, 102, 101', 102') and which, at least in sections, runs on the wall (106, 106') along a circumferential direction directed around the mounting direction (A).

8. Apparatus (1) according to Claim 7, **characterized in that** the groove (103, 103') runs in an encircling manner in circular-ring-shaped form on the wall (106, 106'), which extends around the mounting direction (A), of the dispensing device (100, 100').

9. Apparatus (1) according to one of the preceding claims, **characterized by** a dosing device (11) for the automatic dosing of the amount of liquid medium (M) to be supplied via the dispenser (10).

10. Method for coating a component (20) with a liquid medium (M), in which
- the component (20) is mounted, by way of a section (200) which is to be coated, on a dispenser (10) of an apparatus (1) for coating the component (20),
- the liquid medium (M) is applied to the section (200) to be coated of the component (20) by means of the dispenser (10), and
- wherein the dispenser (10) has at least one dispensing device (100, 100') onto which the component (20) is mounted in a mounting direction (A) and which, at least in sections, extends longitudinally along the mounting direction (A), and the dispensing device (100, 100') has a first end (100A, 100A') and a second end (100B, 100B'), wherein
- the liquid medium (M) for applying to the section (200) to be coated of the component (20) is supplied in the region of the first end (100A, 100A') by means of the dispensing device (100, 100'), at least a part of the liquid medium (M) is suctioned off from the section (200) to be coated of the component (20) by means of a suction device (12) of the apparatus (1) for coating the component (20), and
- by means of the suction device (12), a negative pressure is generated in the region of the second end (100B, 100B') in order to suction the liquid medium (M) at least partially from the region of the first end (100A, 100A') to the second end (100B, 100B'), such that the liquid medium (M), suctioned by the suction device (12), flows along the component (20) to be coated from the first end (100A, 100A') of the dispensing device (100, 100') to the second end (100B, 100B') of the dispensing device (100, 100'), and a liquid film is applied to the section (200) to be coated of the component (20) .

11. Method according to Claim 10, **characterized in that**,
- in a first step, the liquid medium (M) for applying to the section (200) to be coated of the component (20) is supplied in the region of the first end (100A, 100A') by means of the dispensing device (100, 100'), and,
- in a second step, by means of the suction device (12), a negative pressure is generated in the region of the second end (100B, 100B') in order to suction the liquid medium (M) at least partially from the region of the first end (100A, 100A') to the second end (100B, 100B').

## Revendications

1. Dispositif (1) destiné à mouiller un composant technique médical (20) avec un milieu fluide (M), comprenant
- un distributeur (10) destiné à appliquer le milieu fluide (M) sur une portion (200) à mouiller du composant (20) et
- un dispositif d'aspiration (12) pour aspirer au moins une partie du milieu fluide (M) de la portion (200) à mouiller du composant (20),
**caractérisé en ce que**
le distributeur (10) présente au moins un dispositif d'alimentation (100, 100') au niveau duquel peut être positionné un composant (20) dans une direction de positionnement (A) et qui s'étend au moins en partie longitudinalement le long de la direction de positionnement (A) et le dispositif d'alimentation (100, 100') présente une première extrémité (100A, 100A') et une deuxième extrémité (100B, 100B'),
- le dispositif d'alimentation (100, 100') étant réalisé pour acheminer, dans la région de la première extrémité (100A, 100A'), le milieu fluide (M) à appliquer sur la portion (200) à mouiller du composant (20), et
- le dispositif d'aspiration (12) étant réalisé pour produire une dépression dans la région de la deuxième extrémité (100B, 100B'), afin d'aspirer le milieu fluide (M) au moins en partie hors de la région de la première extrémité (100A, 100A') vers la deuxième extrémité (100B, 100B'), de sorte que le milieu fluide (M), aspiré par le dispositif d'aspiration (12), s'écoule le long du composant (20) à mouiller depuis la première extrémité (100A, 100A') du dispositif d'alimentation (100, 100') jusqu'à la deuxième extrémité (100B, 100B') du dispositif d'alimentation (100, 100') et qu'un film de liquide soit appliqué sur la portion (200) à mouiller du composant (20).

2. Dispositif (1) selon la revendication 1, **caractérisé en ce que** le dispositif d'alimentation (100) est réalisé par un tourillon au niveau duquel le composant (20) peut être positionné avec une ouverture (201) pour mouiller en partie une paroi intérieure (202) du composant (20).

3. Dispositif (1) selon la revendication 2, **caractérisé en ce que** le dispositif d'alimentation (100) sous la forme du tourillon, dans l'état positionné du composant (20), est en contact avec la portion (200) à mouiller de la paroi intérieure (202) du composant (20) .

4. Dispositif (1) selon la revendication 1, **caractérisé en ce que** le dispositif d'alimentation (100') est réalisé par une douille dans laquelle le composant (20) avec la portion (200) à mouiller peut être enfiché pour mouiller en partie une paroi extérieure (203) du composant (20) .

5. Dispositif (1) selon la revendication 4, **caractérisé en ce que** le dispositif d'alimentation (100) sous la forme de la douille, dans l'état enfiché du composant (20), s'applique à plat contre la portion (200) à mouiller de la paroi extérieure (203) du composant (20) .

6. Dispositif (1) selon l'une quelconque des revendications précédentes, **caractérisé en ce que** le distributeur présente deux dispositifs d'alimentation (100, 100'), dont
- un premier dispositif d'alimentation (100) est réalisé par un tourillon au niveau duquel le composant (20) peut être positionné avec une ouverture (201) pour mouiller en partie une paroi intérieure (202) du composant (20), et
- un deuxième dispositif d'alimentation (100') est réalisé par une douille dans laquelle le composant (20) avec la portion (200) à mouiller peut être enfiché pour mouiller en partie une paroi extérieure (203) du composant (20) .

7. Dispositif (1) selon l'une quelconque des revendications précédentes, **caractérisé en ce que** l'au moins un dispositif d'alimentation (100, 100') présente un canal (101, 102, 101', 102') pour acheminer le milieu fluide (M) à la région de la première extrémité (100A, 100A') du dispositif d'alimentation (100, 100'), le dispositif d'alimentation (100, 100') présentant, au niveau d'une paroi (106, 106') qui, dans l'état positionné du composant (20), est tournée vers la portion (200) à mouiller du composant (20), une rainure (103, 103') qui est en liaison fluidique avec le canal (101, 102, 101', 102') et s'étend au moins en partie le long d'une direction périphérique orientée autour de la direction de positionnement (A) au niveau de la paroi (106, 106').

8. Dispositif (1) selon la revendication 7, **caractérisé en ce que** la rainure (103, 103') s'étend sous forme annulaire circulaire au niveau de la paroi (106, 106') du dispositif d'alimentation (100, 100') s'étendant autour de la direction de positionnement (A).

9. Dispositif (1) selon l'une quelconque des revendications précédentes, **caractérisé par** un dispositif de dosage (11) pour le dosage automatique de la quantité du milieu fluide (M) devant être acheminé par le biais du distributeur (10).

10. Procédé pour mouiller un composant (20) avec un milieu fluide (M), dans lequel
- le composant (20) est positionné avec une portion (200) à mouiller au niveau d'un distributeur (10) d'un dispositif (1) destiné à mouiller le composant (20),
- au moyen du distributeur (10), le milieu fluide (M) est appliqué sur la portion (200) à mouiller du composant (20) et
- le distributeur (10) présentant au moins un dispositif d'alimentation (100, 100') au niveau duquel le composant (20) est positionné dans une direction de positionnement (A) et qui s'étend au moins en partie longitudinalement le long de la direction de positionnement (A), et le dispositif d'alimentation (100, 100') présentant une première extrémité (100A, 100A') et une deuxième extrémité (100B, 100B'),
- le milieu fluide (M) destiné à être appliqué sur la portion (200) à mouiller du composant (20) étant acheminé au moyen du dispositif d'alimentation (100, 100') dans la région de la première extrémité (100A, 100A'),
**caractérisé en ce qu'**au moyen d'un dispositif d'aspiration (12) du dispositif (1) destiné à mouiller le composant (20), au moins une partie du milieu fluide (M) est aspirée de la portion (200) à mouiller du composant (20), et
- au moyen du dispositif d'aspiration (12) dans la région de la deuxième extrémité (100B, 100B') une dépression est produite, afin d'aspirer le milieu fluide (M) au moins en partie depuis la région de la première extrémité (100A, 100A') vers la deuxième extrémité (100B, 100B') de sorte que le milieu fluide (M), aspiré par le dispositif d'aspiration (12), s'écoule le long du composant (20) à mouiller depuis la première extrémité (100A, 100A') du dispositif d'alimentation (100, 100') vers la deuxième extrémité (100B, 100B') du dispositif d'alimentation (100, 100') et qu'un film de liquide soit appliqué sur la portion (200) à mouiller du composant (20).

11. Procédé selon la revendication 10, **caractérisé en ce que**
- au moyen du dispositif d'alimentation (100, 100'), dans une première étape, le milieu fluide (M) destiné à être appliqué sur la portion (200) à mouiller du composant (20) est acheminé dans la région de la première extrémité (100A, 100A') et
- au moyen du dispositif d'aspiration (12), dans une deuxième étape, une dépression est produite dans la région de la deuxième extrémité (100B, 100B') afin d'aspirer le milieu fluide (M) au moins en partie de la région de la première extrémité (100A, 100A') vers la deuxième extrémité (100B, 100B').
